# EUROPEAN PATENT SPECIFICATION

(11) **EP 1 771 156 B1**
(45) Date of publication and mention of the grant of the patent: **11.08.2010**
(21) Application number: 05759293.3
(22) Date of filing: 13.06.2005
(51) Int. Cl.: A61K 9/127, A61K 31/7048

(54) **STEROL ENRICHED MIXED LAMELLARITY AMPHOTERICIN INTERCALATING LIPOSOMES IN SALINE AND THE PROCESS FOR THEIR PREPARATION**
STEROL-ANGEREICHERTE MULTILAMELLARE AMPHOTERICIN-ENTHALTENDE LIPOSOMEN IN KOCHSALZLÖSUNG UND HERSTELLUNGSVERFAHREN
LIPOSOMES INTERCALANTS D'AMPHOTERICINE A LAMELLARITE MIXTE ENRICHIS DE STEROL EN SOLUTION SALINE ET PROCEDE POUR LEUR PREPARATION

(30) Priority: 11.06.2004 IN DE11132004
(43) Date of publication of application: 11.04.2007
(73) Proprietor: Verma, Jitendra, Nath, Gurgaon 122 002 HR (IN); Verma, Lily, Gurgaon 122 002 HR (IN); Tripathi, Krishan, Kumar, Andrewsganj New Delhi 110 049 (IN)
(72) Inventor: Verma, Jitendra, Nath, Gurgaon 122 002 HR (IN); Verma, Lily, Gurgaon 122 002 HR (IN); Tripathi, Krishan, Kumar, Andrewsganj New Delhi 110 049 (IN)
(74) Representative: Jorio, Paolo
(86) International application number: PCT/IN2005/000193
(87) International publication number: WO 2005/120460

(56) References cited:
- GB-A- 2 134 869
- US-A- 4 766 046
- SZOKA F C JR ET AL: "EFFECT OF LIPID COMPOSITION AND LIPOSOME SIZE ON TOXICITY AND IN-VITRO FUNGICIDAL ACTIVITY OF LIPOSOME-INTERCALATED AMPHOTERICIN B" ANTIMICROBIAL AGENTS AND CHEMOTHERAPY, vol. 31, no. 3, 1987, pages 421-429, XP002346882 ISSN: 0066-4804
- MEHTA R ET AL: "Liposomal amphotericin B is toxic to fungal cells but not to mammalian cells." BIOCHIMICA ET BIOPHYSICA ACTA. 14 MAR 1984, vol. 770, no. 2, 14 March 1984 (1984-03-14), pages 230-234, XP002346883 ISSN: 0006-3002 cited in the application
- TREMBLAY C ET AL: "EFFICACY OF LIPOSOME INTERCALATED AMPHOTERICIN B IN THE TREATMENT OF SYSTEMIC CANDIDIASIS IN MICE" ANTIMICROBIAL AGENTS AND CHEMOTHERAPY, vol. 26, no. 2, 1984, pages 170-173, XP002346884 ISSN: 0066-4804
- MURRAY ET AL.: "Clinical and Experimental Advanced in Treatment of Visceral Leishmaniasis" ANTIMICROBIAL AGENTS AND CHEMOTHERAPY, no. Aug., 2001, pages 2185-2197,

## Description

### FIELD OF INVENTION:

The invention relates to ergosterol enriched mixed lamellarity Amphotericin intercalating liposomes in saline and the process of preparing it.

### BACKGROUND OF THE INVENTION:

Leishmaniasis is caused by macrophage resident intracellular protozoan parasites *Leishmania donovani, Leishmania infantum and Leishmania chagasi.* Parasite transmission occurs through the bite of infected female phlebotomine sandfly. Sandfly obtains infected blood while sucking the blood from their parasite carrier hosts. Four different forms of Leishmaniasis with different clinical manifestations occur in humans. Of these different forms, Visceral Leishmaniasis (VL), which is also known as kala-azar is the most severe and has a mortality rate of almost 100% if not treated. Frequent bouts of fever, weight loss, enlargement of spleen and liver accompanied with anemia are characteristic signs and symptoms of Visceral Leishmaniasis.

Amphotericin B is a polyene antibiotic produced by soil bacteria *Streptomyces nodosus.* Amphotericin B is known to be the most potent antifungal drug since its discovery in 1950s. Later it was found to be an effective treatment for kala-azar also (Trans Royal Soc. Trop. Med. Hyg. 1963; 57: 266-268).

Unlike alarming incidents of resistance against commonly used and first line drugs viz. Sodium Stibogluconate and Pentamidine, Amphotericin B is free of drug resistance problems (CP Thakur et.al. 1993. The National Medical Journal of India; 6: 57-60 and CP Thakur et.al. 1993. Indian Journal of Medical Research; 97: 170-175).
Despite being an effective anti-lieshmanial drug, amhotericin B has been used cautiously only as second line drug due to it's infusion related adverse effects and toxicities, predominantly nephrotoxicity, cardio-toxicity and neuro-toxicity. As no other drug could replace the broad spectrum and the potency of amphotericin B, understandably, various strategies have been experimented and even practiced with limited success to overcome dose related toxicities of amphotericin B.

The most unscientific, ambiguous, unacceptably inconsistent, and therefore least practiced way to overcome amphotericin B related toxicity is to heat, cool and then administer conventional amphotericin B. There have been clinical trials for evaluation of benefits of combining Intralipid (an IV lipid suspension) with conventional amphotericin B as a substitute for true lipid formulations of amphotericin B (Dupont B. 2002, J. Antimicrobial Chemotherapy; 49, Suppl. S1, 31-36).

In one such comparative study on 82 children employing amphotericin B deoxycholate suspension alone and its mixture with Intralipid, no benefit of mixing was found in improving safety or tolerance of amphotericin B (Nath, CE et.al. 1999, Antimicrobial Agents and Chemotherapy; 43: 1417-1423). Both forms of amphotericin B were comparatively evaluated in another study on adults patients, and the observations revealed that there is no significant difference in nephrotoxicity in the groups administered amphotericin B with or without Intralipid (Nucci, M et.al.1999, Antimicrobial Agents and Chemotherapy; 43:1445-1448).

Besides the lack of advantage of Intralipid admixture, there are significant problems associated with administration of amphotericin B-Intralipid mixture. These are: lower therapeutic activity, thrombocytopenia, liver function abnormalities, cholestasis and pulmonary toxicity (Deray,G. 2002, J. Antimicrobial Chemotherapy; 49, Suppl. S1, 37-41).

Yet another alternative usually considered for reducing toxic effects of amphotericin B is infusion at slower rate. Although, a prospective study concluded that slower infusion rate did not reduce Amphotericin B toxicity (Ellis, M.E. 1992, Antimicrobial Agents and Chemotherapy; 36: 172-179), in patients with renal insufficiency, rapid infusion is advised to be avoided to overcome severe hyperkalaemia and potentially fatal arrhythmia (Bell, N.H. et.al.1962, American Journal of Medicine; 340:64-69 and Craven P.C.1985, Antimicrobial Agents and Chemotherapy; 27:868-871). The toxicities of amphotericin B are so critical that with very limited and incremental advantages also, slow infusion of all formulations of amphotericin B has become a standard practice.

Adverse effects of amphotericin B have been successfully overcome, however, by lipid formulations of amphotericin B (Sunder S and Murray HW, 1996:173: 762-765 and Seaman et. al. 1995; 21: 188-193). Liposomal amphotericin B is now established as the most effective and safe drug which is also free of drug resistance problems.

Strategic suitability of liposomal formulations of amphotericin B for the treatment of Visceral Leishmaniasis emanates from several factors including factors such as leishmania resides in the scavenger cells such as macrophages, liposomes are rapidly taken up by the macrophages, amphotericin B is an effective anti-leishmanial drug, and amphotericin B can be formulated as liposomal preparation. All of these facilitate targeted delivery of liposomal amphotericin B to the desired site of anti-parasitic action and reduce toxicities of amphotericin B. Additionally liposomes offer an amplification effect through concentrated encapsulation of numerous molecules of drug in each liposome particle, which are delivered at the site of action (Gregoriadis G, 1995. Engineering liposomes for drug delivery: Progress and Problems. TIBTECH; 13: 527-537). Prevention of toxicity by encapsulating amphotericin B in liposomes has made liposomal amphotericin B the best option for the treatment of Visceral Leishmaniasis.

The critical lipid constituent for minimizing amphotericin B toxicity of formulations for use in treatment of systemic mycosis, are sterols. Of the sterols available for formulations, cholesterol, cholesterol succinate and cholesteryl sulphate have been used in experimental and/or commercial amphotericin B lipid preparations. Formulations of amphotericin B constituted of ergosterol with certain phospholipids have been reported to cause lysis of red cells *in-vitro* and thus believed to be unsuitable (Mehta, R. et.al. 1984, Biochimica et Biophysica Acta; 770: 230-234).

Unsuccessful attempts with limited number of phospholipid combination and ergosterol has prevented further efforts on development of ergosterol containing amphotericin B formulations (New, P.R.C. et.al.1981, J. Antimicrobial Agents and Chemotherapy;8: 371-381 and Graybill, J.R. et.al. 1982, J. Infectious Diseases; 145: 748-752)

Cytolytic action of amphotericin B is mediated through the interaction of amphotericin B with the sterols present in the cells. The drug has stronger affinity to the ergosterol, a major constituent of fungal cell membranes (Szoka, F.C. and Tang, M. 1993, J. Liposome Research; 3:363-375) and precursor of ergosterol present in kala-azar causing parasite *Leishmania* (Meyerhoff, A.1999, Clinical Infectious Diseases; 28: 42-48) than to cholesterol, a sterol present in most mammalian cell membranes. Binding of amphotericin B with sterols in the cell membranes, results in pore formation, causing cytolytic action of the drug. Both, the therapeutic antimicrobial action and toxicity to the mammalian cells are caused by the same mechanism.

In order to reduce toxicity to the host cell achieved by targeting the pathogen and avoidance of the mammalian host, cholesterol containing liposomes emerged as matchless strategy for the treatment of systemic mycosis with even aggressive treatment with high doses up to 15mg/kg body wt/day. For such aggressive treatment it would seem that a better approach would be to administer formulations with higher concentration of amphotericin B. Apparent concerns of toxicities expected from concentrated preparations of amphotericin B, have restricted all preparations of amphotericin B to 1mg/ml with recommendations to infuse slowly.

Such concerns of amphotericin B toxicity assume more significance for treatment of leishmaniasis caused by macrophage resident intracellular parasite with liposomal amphotericin B. Liposomes are known to be rapidly phagocytosed in macrophages and will obviously result in concentration of liposome encapsulated amphotericin B in the macrophages. To formulate liposomes with higher concentration of amphotericin B without causing toxicity required strategies, which are not yet reported.

Understanding of sterol synthesis is important for rational designing of quick, effective and specific treatment of leishmaniasis using liposomal amphotericin B. Lipids account for 15% of the dry weight of the leishmanial cells (Meyer and Ilolz, 1966, J. Biol. Chem. 24: 5000-5007). Metabolism of lipids is crucial for several vital physiological processes and effect parasite's survival. Ergosterol or its precursor episterol is synthesized *de novo* from AcetylCoA to evalonate to Sqalene to Lanosterol followed by four more steps to finally Ergosterol ( Coppens and Courtoy. 1995, Mol. Bichem. Parasitol. 73,179-188 and Patent No. US 6,403,576B1).

It is pertinent to note that Leishmania require cholesterol for their sustenance and fulfill their requirement by salvaging cholesterol from their host macrophage cells. Effective drug designing prudently shall involve adversely effecting survival of Leishmania by depriving Leishmania of their cholesterol requirement.

Delivery of cholesterol containing liposomes through phagocytic action of reticuloendothelial cells to some extent negates the benefits of targeted delivery of amphotericin B in the macrophage, the home of Leishmania.

Administration of 20-21 mg of Amphotericin B in shorter durations also results in administration of sucrose undesirable in diabetics. Mixture of conventional Amphotericin B with 0.9 % Sodium Chloride is known to result in precipitation of Amphotericin B (Martindale, p315). Furthermore, mono and disaccharides such as glucose, mannitol, trehalose, sucrose and lactose are also reported to render stability to liposomal formulations (US Patent No.5,180,713). These observations have argued against use of Sodium Chloride solution for suspension of liposomal amphotericin B and directions for use advise against use of saline (AmBisome product information).

US-A-4766046 discloses liposomes comprising Amphotencin B, chotesterol, phosphatidylglycerol and tocopherol. The liposomes are suspended in aqueous medium comprising a buffer solution, such as PBS.

### SUMMARY OF THE INVENTION:

The main object of the invention is to provide a ergosterol enriched mixed lamellarity amphotericin intercalating liposomes for treatment of infections for targeted delivery of liposomal amphotericin B thereby reducing toxicities of amphotericin B.

Another object of the invention is to make liposomal amphotericin B with suitable replacement of cholesterol. Cholesterol is normally used for preventing leakiness of liposomal drugs / vaccines and in case of amphotericin B formulations cholesterol is used for targeting and minimizing drug toxicities. Cholesterol, however, supports leishmania survival inside the host , therefore should not be taken as constituent of formulation.

Yet another object of the invention is to deliver higher concentration of amphotericin B to the target area without causing toxicity.

Another object of invention is to sonicate liposomal pharmaceuticals before administration to increase the plasma half-life of the liposomal drug for facilitating better bio-distribution in the body.

Yet another object of the instant invention is to provide a sugar free composition containing liposomal amphotericin B and therefore freely usable by diabetics

To achieve to aforementioned objects the instant invention provides for ergosterol enriched mixed lamellarity Amphotericin intercalating liposomes according to claim 1. There is high Amphotericin B concentration in liposomes.

The sterol is ergosterol with or without cholesterol. The sterol are in combination with phospholipid. The said phospholipid is phosphatidylcholine. The ergosterol constitutes 50% of the total lipid of the liposome. One example of the preferred ratio of phosphatidylcholine, ergosterol and amphotorecin B is 5 : 2 : 1.

Sonication before administration increases plasma half-life and provides for better bio distribution.

### DETAILED DESCRIPTION OF THE INVENTION:

Choice of lipid constituents of liposomes particularly sterol is critical to prudent designing of potently effective and safe anti-leishmanial drug. Inclusion of appropriate sterol is necessary to prevent binding of liposomal amphotericin B to mammalian host cells. Delivery of cholesterol in macrophage home of Leishmania through the phagocytic uptake of cholesterol containing liposomal amphotericin B negates optimal benefits of targeted delivery of amphotericin B. Another sterol option of ergosterol in combination with certain lipids in liposomes as outlined above has been shown to cause toxicity to red blood cells *in*-*vitro*. This invention was taken up to design cholesterol free and suitable sterol containing liposomal amphotericin B.

Ergosterol with or without cholesterol and in combination with phospholipids such as phosphatidylcholine irrespective of their fatty acid compositions from either synthetic or from natural sources such as Soy in a range of ratios was found suitable for formulating liposomal amphotericin B which is safe and potently effective against infections such as Leishmania. Amphotericin B concentration range was used between 1-15 mg /ml of the final preparation. The lipids i.e. phospholipids preferably phosphatidylcholine and ergosterol are taken in such ratios where ergosterol constitutes up to 50 molar % of the total lipids of the liposomal formulations. One preferred formulation for example is PC: Ergosterol : Amphotericin B in the molar ratio of 5:2:1 in aqueous medium.

Amphotericin B is well documented to precipitate in saline. Amphotericin B in liposomes of phoshopholipid compositions reported herein above are stabilized and no precipitation occurs even on long term storage. The invention permits replacement of dextrose with saline without buffers. One preferred example is to use 0.9 % Sodium Chloride for suspension of liposomal amphotericin B. Such liposomal formulations are stable both in suspension or when stored after lyophilization.

Alternatively, lyophilized Liposomal Amphotericin B may be reconstituted in aqueous solutions such as 0.9% Sodium Chloride.

The process of preparing the liposomal Amphotericin B is explained but not limited by the following examples.

Liposomes can be manufactured by employing a variety of methods either alone or in combination (Szoka, F. Jr. and Papahadjopoulos, D., 1980. Ann. Rev. Biophys. Bioeng. 9: 467-508).

Example 1. Methanolic solution of the Liposomal constituents are evaporated to give a film of the constituents. This film is hydrated using aqueous solutions and multilamellar Liposomes are produced by sheering of the film.

Example 2. Methanolic solution of the Liposomal constituents are processed in Spray Dryer and then hydrated and reconstituted in aqueous medium of choice to give mixed lamellar Liposomal preparation.

Example 3. Methanolic solution of the Liposomal constituents are evaporated to give a film of the constituents, aqueous solution of choice is added and then subjected to Sonication resulting predominantly into Unilamellar Liposomal Preparation.

### Bio-therapeutic properties

Ergosterol-PC Liposomes with higher concentration of Liposomes are well tolerated and have been found to be safe with respect to both chronic and acute toxicity. These Liposomal formulations with up to 15mg Amphotericin B/ml of preparations could be administered without any infusion related adverse effect. LDsoof these formulations is higher than reported for Cholesterol-PC liposomal amphotericin B.

Nephrotoxicity was determined by measuring changes in serum creatinine value at various time periods after the administration of this drug. Serum Creatinine value remained significantly unaltered for up to the administration of complete dosage i.e. 21mg/ kg body weight required for complete treatment of Leishmaniasis.

Leishmania are completely cleared using any of the concentrated Amphotericin B encapsulated Ergosterol-PC Liposomes after administration of 21mg of Amphotericin B in liposomes reported herein. Only in few cases, additional dose of Liposomal Amphotericin B was required for complete parasite clearance.

To support the findings, a tolerance study was conducted in mice using the sterol composition of the instant invention prepared as described herein. A higher concentration of Amphotericin B of up to 10mg. per ml. was administered. The tolerance studies indicate that 100 mg Amphotericin B /kg as a single dose in the ergosterol for the liposomal formulation was well tolerated.

### REFERENCES:

Nath, CE et.al. 1999, Antimicrobial Agents and Chemotherapy; 43: 1417-1423
Ellis, M.E. 1992, Antimicrobial Agents and Chemotherapy;36: 172-179
Bell, N.H. et.al.1962, American Journal of Medicine; 340:64-69
Craven P.C.1985, Antimicrobial Agents and Chemotherapy; 27:868-871

## Claims

1. ErgoSterol enriched mixed lamellarity Amphotericin intercalating liposomes in aqueous suspension wherein the concentration of Amphotericin B is 1 to 15 mg/ml and the aqueous suspension is a suspension in an aqueous solution of 0.9% Sodium Chloride without a buffer for use in treatment of infections.

2. ErgoSterol enriched mixed lamellarity Amphotericin intercalating liposomes as claimed in claim 1 wherein the ergosterol is in combination with phospholipid.

3. ErgoSterol enriched mixed lamellarity Amphotericin intercalating liposomes as claimed in claim 2 wherein the said phospholipid is phosphatidylcholine.

4. ErgoSterol enriched mixed lamellarity Amphotericin intercalating liposomes as claimed in claim 1 wherein ergosterol constitutes up to 50 molar % of the total lipid of the liposome.

5. ErgoSterol enriched mixed lamellarity Amphotericin intercalating liposomes as claimed in claims 1 to 3 wherein the ratio of phosphatidylcholine, ergosterol and Amphotorecin B is 5 : 2 : 1.

6. ErgoSterol enriched mixed lamellarity Amphotericin intercalating liposomes as claimed in claim 1 wherein the infection is due to intracellular protozoan parasites including Leishmania.

7. ErgoSterol enriched mixed lamellarity Amphotericin intercalating liposomes as claimed in claim 1 wherein the said composition is sonicated during manufacturing and before administration for increasing plasma half-life and better bio-distribution.

## Patentansprüche

1. Ergosterol-angereicherte, Amphotericin interkalierende Liposome gemischter Lamellarität in wässriger Suspension, wobei die Konzentration von Amphotericin B 1 bis 15 mg/ml beträgt und die wässrige Suspension eine Suspension in einer wässrigen Lösung von 0,9 % Natriumchlorid ohne einen Puffer ist, zur Verwendung bei der Behandlung von Infektionen.

2. Ergosterol-angereicherte, Amphotericin interkalierende Liposome gemischter Lamellarität wie in Anspruch 1 beansprucht, wobei das Ergosterol mit Phospholipid in Kombination ist.

3. Ergosterol-angereicherte, Amphotericin interkalierende Liposome gemischter Lamellarität wie in Anspruch 2 beansprucht, wobei das Phospholipid Phosphatidylcholin ist.

4. Ergosterol-angereicherte, Amphotericin interkalierende Liposome gemischter Lamellarität wie in Anspruch 1 beansprucht, wobei Ergosterol bis zu 50 Mol-% des Gesamtlipids des Liposoms ausmacht.

5. Ergosterol-angereicherte, Amphotericin interkalierende Liposome gemischter Lamellarität wie in den Ansprüchen 1 bis 3 beansprucht, wobei das Verhältnis von Phosphatidylcholin, Ergosterol und Amphotericin B 5 : 2 : 1 beträgt.

6. Ergosterol-angereicherte, Amphotericin interkalierende Liposome gemischter Lamellarität wie in Anspruch 1 beansprucht, wobei die Infektion infolge von intrazellulären Protozoen-Parasiten einschließlich Leishmania besteht.

7. Ergosterol-angereicherte, Amphotericin interkalierende Liposome gemischter Lamellarität wie in Anspruch 1 beansprucht, wobei die Zusammensetzung während der Herstellung und vor der Verabreichung beschallt wird, um die Halbwertszeit des Plasmas zu erhöhen und die biologische Verteilung zu verbessern.

## Revendications

1. Liposomes intercalants de l'amphotéricine à lamellarité mixte enrichis en ergostérol, en suspension aqueuse, dans lesquels la concentration de l'amphotéricine B est de 1 à 15 mg/ml et la suspension aqueuse est une suspension dans une solution aqueuse de 0,9% de chlorure de sodium sans tampon pour une utilisation dans le traitement des infections.

2. Liposomes intercalants de l'amphotéricine à lamellarité mixte enrichis en ergostérol tels que revendiqués dans la revendication 1, dans lesquels l'ergostérol est en combinaison avec un phospholipide.

3. Liposomes intercalants de l'amphotéricine à lamellarité mixte enrichis en ergostérol, tels que revendiqués dans la revendication 2, dans lesquels ledit phospholipide est la phosphatidylcholine.

4. Liposomes intercalants de l'amphotéricine à lamellarité mixte enrichis en ergostérol, tels que revendiqués dans la revendication 1, dans lesquels l'ergostérol constitue jusqu'à 50 moles % des lipides totaux du liposome.

5. Liposomes intercalants de l'amphotéricine à lamellarité mixte enrichis en ergostérol, tels que revendiqués dans les revendications 1 à 3, dans lesquels le rapport phosphatidylcholine, ergostérol et amphotéricine B est de 5 : 2 : 1.

6. Liposomes intercalants de l'amphotéricine à lamellarité mixte enrichis en ergostérol, tels que revendiqués dans la revendication 1, dans lesquels l'infection est due à des parasites protozoaires intracellulaires comprenant la Leishmania.

7. Liposomes intercalants de l'amphotéricine à lamellarité mixte enrichis en ergostérol, tels que revendiqués dans la revendication 1, dans lesquels ladite composition est traitée par des ultrasons pendant la fabrication et avant l'administration pour augmenter la demi-vie du plasma et assurer une meilleure biodistribution.
